# EUROPEAN PATENT SPECIFICATION

(11) **EP 4 228 727 B1**
(45) Date of publication and mention of the grant of the patent: **16.09.2026**
(21) Application number: 21806343.6
(22) Date of filing: 13.10.2021
(51) Int. Cl.: A61M 16/00, A61M 16/12, A61M 16/20, A61M 16/22, A61B 5/08, A61M 16/08, A61M 16/10

(54) **CPAP KIT TO SUPPORT BREATHING**
CPAP-KIT ZUR UNTERSTÜTZUNG DER ATMUNG
KIT CPAP POUR L'ASSISTANCE RESPIRATOIRE

(30) Priority: 13.10.2020 IT 202000024079
(43) Date of publication of application: 23.08.2023
(73) Proprietor: Politecnico Di Torino, 10129 Torino (IT); APR S.r.l., 10064 Pinerolo (Torino) (IT); Olivieri, Carlo, 28100 Novara (IT)
(72) Inventor: AUDENINO, Alberto, 10129 Torino (IT); RAPARELLI, Terenziano, 10129 Torino (IT); MORBIDUCCI, Umberto, 10129 Torino (IT); CAVAGLIA', Marco, 10129 Torino (IT); IVANOV, Alexander, 10129 Torino (IT); ROMITI, Armando, 10064 Pinerolo (Torino) (IT); ROMITI, Andrea, 10064 Pinerolo (Torino) (IT); FERRARA, Marino, 10064 Pinerolo (Torino) (IT); CHIESA, Alessandro, 10064 Pinerolo (Torino) (IT); OLIVIERI, Carlo, 28100 Novara (IT)
(74) Representative: Mola, Edoardo
(86) International application number: PCT/IB2021/059423
(87) International publication number: WO 2022/079642

(56) References cited:
- EP-A1- 3 912 665
- EP-A1- 4 157 409
- EP-A2- 0 894 506
- WO-A1-2015/048766
- GB-A- 2 548 548
- US-A- 5 590 644
- US-A1- 2012 174 926
- US-A1- 2019 167 928
- US-B2- 9 238 115

## Description

### Technical Field

The present invention refers to a kit to support the breathing of a non-anesthetized individual with respiratory diseases but is able to breathe spontaneously. In particular, breathing occurs in CPAP mode, i.e. Continuous Positive Airway Pressure i.e. the kit works at positive pressure with respect to ambient pressure and ideally constant both when the user inhales and when the user exhales, in particular between values of 5 and 25 cmH2O, more preferably between values of 5 and 20 cmH2O and controlled by a pressometric ventilation system.

### STATE OF THE ART

It is known to support breathing by delivering positive pressure flows with relatively high flow rates of pressurized gas in order to remove exhaled carbon dioxide. However, in addition to high consumption of medical gases, this also causes the generation of noise and alters the humidity of the breathed gas, reducing the comfort of the user.

Closed circuits equipped with a CO₂ absorber commonly used for anesthesia are also known, however the inhalation of fresh gases and possibly anesthetic gas takes place through a pressovolumetric machine in different conditions than the CPAP mode in spontaneous breathing i.e. with a pressometric fan. During the supply of an anesthetic substance, not only the quantity of active principle suplied is monitored but, due to the user loss of consciousness, it is essential to control both the volumes and the frequency of gases provided by means of suitable pressure oscillations in the closed circuit. This is valid both in the case of anesthesia with assisted ventilation and in that with controlled ventilation.

Document WO-A1-2015048766 describes a unit for suppling Heliox comprising a closed breathing circuit and a first and a second re-breathing cylinder of respriatory gas; wherein the internal pressure fluctuates due to the patient's breathing and cannot exceed peaks of 4 cmH₂O. Air circulation is guaranteed by a turbine in line with the circuit. This is useful to help the user overcome the fluid-dynamic friction of the circuit during spontaneous breathing.

### SCOPES AND SUMMARY OF THE INVENTION

The object of the present invention is to provide a kit to support spontaneous lung respiration without anesthesia capable of solving the problems indicated above and, in addition, reducing the flow rate and in particular the consumption of oxygen from an additional source to that of the air, avoid the generation of aerosols, which carry contagious pathogens, and adapting to existing devices with a relatively low cost.

The object of the present invention is achieved by means of a breathing support kit according to claim 1 comprising: a first inlet for receiving pressurized air; a second inlet for receiving oxygen; a section where the air from the first inlet and the oxygen from the second inlet converge; a closed air circuit comprising the first node, a wearable breathing element applicable at least on the nose and mouth of a patient and configured to receive from the circuit a mixture of air and oxygen coming from the section via a first circuit branch and to transfer the exhaled air enriched with carbon dioxide to the circuit via a second circuit branch, an air treatment filter along the second circuit branch to reduce or eliminate carbon dioxide in the second circuit branch, a valve unit configured for force a one-way air circulation from the section to reach the treatment filter via the breathing wearable element, a pressure sensor and a pressure regulator configured to adjust the pressure in the closed circuit based on a pressure sensor signal to obtain a constant CPAP pressure in the closed loop during breathing spontaneous by the user in the wearable breathing element.

In this way, the kit has a closed circuit for enriched air which includes a partial or full face mask to be applied to the individual's face or a ventilation hood/helmet, and thus no aerosols carrying viral, bacterial or, more generally, microbial loads are diffused into the environment surrounding the individual or patient. Furthermore, since it is a closed fluid circuit during spontaneous breathing, only the amount of oxygen breathed and retained by the user, i.e. the oxygen used in the respiratory cycles must be reintegrated, allowing a substantial containment of oxygen consumption. Carbon dioxide is absorbed by the treatment filter. The constant positive CPAP pressure with respect to the atmospheric pressure applied to the air provides an expansion action of the pulmonary alveoli, thus favoring the exchange of oxygen / carbon dioxide with the blood. A simple and highly functional tool is therefore provided to treat users suffering from respiratory diseases and pathogenic contagious respiratory viruses, bringing a respiratory benefit through the opening of the pulmonary alveoli, greatly limiting the consumption of oxygen and the circulation of pathogens. In addition, the closed circuit allows easier conservation of the humidity levels of the air breathed and intrinsically reduces the noise allowing a support to the user with autonomous breathing for long periods with a good overall comfort.

According to a preferred embodiment, the valve unit is configured to open and close to lead the air in the direction of circulation following the respiratory activity of the patient to whom the mask is applied.

According to a preferred embodiment, the kit comprises a fluidic capacity connected in derivation to the first branch to reduce the pressure fluctuations of the air entering the mask.

It is in fact demonstrable that a patient benefits from breathing air at constant or low oscillating pressure, i.e. constant and positive CPAP pressure, which is maintained thanks to the regulating device on the base of the pressure sensor.

According to a preferred embodiment, the kit comprises a carbon dioxide sensor and / or a temperature sensor and / or a humidity sensor and a safety valve configured to open automatically and allow air to enter the circuit on the basis of a signal coming from at least one of said sensors.

Optionally, in addition to opening the safety valve, the kit includes an alarm device activated on the basis of at least one of the sensors. In particular, the pressure sensor detects undesired operating conditions such as excessive variations or inversions of pressure in the circuit. In this way, safety in the event of oxygen mixing malfunction is increased.

Preferably, the kit includes an antiviral / antibacterial or antimicrobial filter in order to purify the air in the closed circuit from pathogens.

According to a preferred embodiment, the kit comprises an electronic control unit programmed to control the quantity of air entering and leaving the first inlet on the basis of at least one operating parameter of the circuit and, therefore, through the first inlet in use there is an exchange of gas volumes also from the inside of the closed circuit towards the outside; during normal operation of the closed circuit, the average flow rate exchanged is zero. It should be noted that the first inlet can be connected to a nitrogen source which, mixed with the inlet oxygen, provides the gas mixture with the desired FiO₂ and the desired positive pressure for the user. This is possible, for example, in a hospital. It is also possible that the first inlet receives pressurized atmospheric air, for example via a fan, preferably centrifugal and speed-controlled. In use, the oxygen consumption during a start-up transient is limited only to the quantity necessary to reach the desired and set FiO₂ in the closed circuit; in steady state operation, i.e. when the set CPAP pressure is reached, this consumption is always equal to the quantity consumed by the patient alone.

Furthermore, via the electronic control, it is possible to increase performance and safety measures. Examples of operating parameters in one or more points of the circuit are, preferably in order of importance, representative of: amount of carbon dioxide (for the user's safety) and / or amount of oxygen and / or temperature and / or humidity. It is also possible to monitor the flow rate circulating in the closed circuit for patient diagnosis. The control unit can be programmed to collect and process the data received from the sensors in order to produce data interpolation according to predefined algorithms to be displayed by connecting to a screen, allowing the operator to intervene as needed. The internal pressure of the circuit can also be controlled by pneumatic control circuits or fluidic devices.

According to a preferred embodiment, the kit comprises an electric fan connected to the first inlet. The electric fan is an embodiment of a source of pressometric pressure and, given the extremely small quantities of air to be integrated in the start-up transient and the ideally zero average flow rate in steady-state operation, this fan can have extremely small construction dimensions e.g. together with its own electric motor it can also fit in the palm of a hand.

In particular, the fan is an electrical fan capable of generating a pressure of less than 30 cm of H₂O. This also allows to make the kit portable, for example by connecting the fan to a rechargeable battery.

According to a preferred embodiment, the kit comprises a portable source of pressurized oxygen connected to the second inlet.

Also disclosed is a method for setting up a device for breathing aid, comprising the steps of:
- providing a kit comprising: a first inlet for receiving pressurized air; a second inlet for receiving oxygen; a section where the air from the first inlet and the oxygen from the second inlet converge; an air circuit comprising: the first node, a first branch configured to adduct a mixture of air and oxygen to the user, a second branch to receive the mixture of air exhaled by the user enriched with carbon dioxide, a treatment filter of the air along the second branch of the circuit to reduce or eliminate carbon dioxide in the second branch and a valve assembly configured to impose air circulation in a single direction starting from the first node to reach the treatment filter via the wearable breathing element
- mounting the kit to a wearable breathing element applicable at least on the nose and mouth of a patient and configured to receive from the circuit a mixture of air and oxygen coming from the first node through the first branch of the circuit and to give the second branch the exhaled air enriched with carbon dioxide;
- controlling the internal pressure of the closed circuit to obtain a constant CPAP pressure during the user's spontaneous breathing in the breathing wearable, using a pressure sensor and a pressure regulator configured to adjust the pressure in the closed circuit based on a signal from the pressure sensor.

In this way, the wearable breathing element can also be treated commercially in a different way from the other components of the kit: for example, the mask can be reusable e.g. after sterilization and the other components of the kit be disposable or the other way around.

Other advantages of the present invention are discussed in the description and cited in the dependent claims.

### BRIEF DESCRIPTION OF THE DRAWINGS

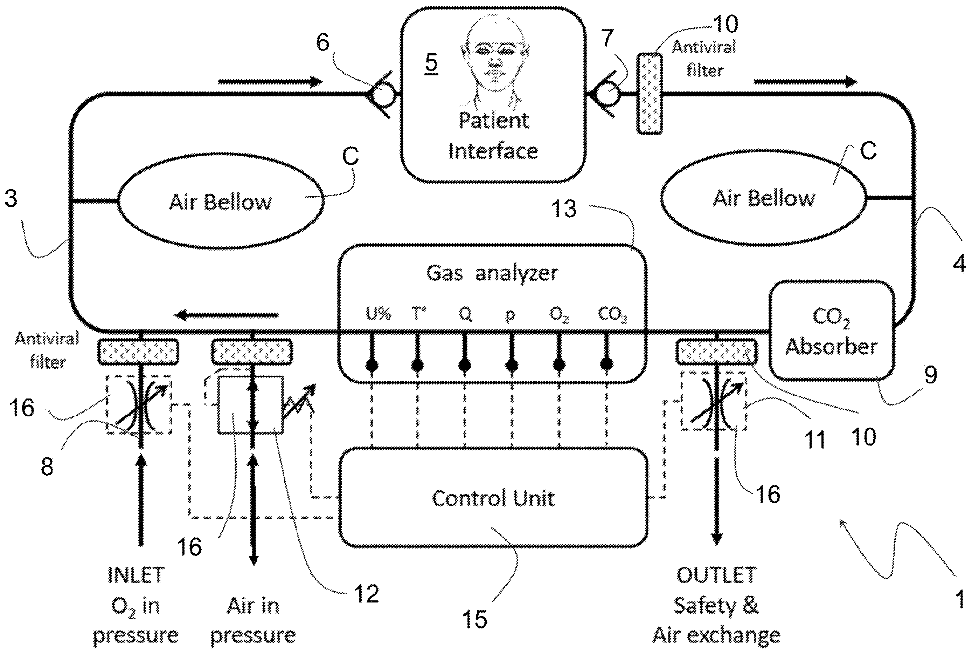

The invention is described below on the basis of non-limiting examples illustrated by way of example in the figure that schematically shows a kit according to the present invention.

### DETAILED DESCRIPTION OF THE INVENTION

The figure schematically shows with 1 as a whole a kit for supporting autonomous breathing without anesthetic gases comprising a closed circuit 2 having an inspiration branch 3, an expiration branch 4 and a wearable breathing element 5 applicable in use at least on the nose and mouth of the patient to receive air richer in oxygen via the inhalation branch 3 and send air richer in carbon dioxide to the exhalation branch 4. Conveniently, a valve unit creates a forced air flow along the circuit to circulate in a single direction, i.e. from the inspiration branch 3 to the expiration branch 4. Preferably, the valve assembly comprises a first non-return valve 6 along the inspiration branch 3 and a non-return valve 7 along the expiration branch 4. According to an embodiment, the breathing wearable 5 is releasably connected to the circuit 2 and the valve assembly 6, 7 can either be on board the breathing wearable element 5, or be carried on the inhalation and exhalation branches 3, 4 when the wearable breathing element 5 is fluidically disconnected from circuit 2.

In order to ensure the best respiratory conditions for a user, the inspiration branch 3 comprises a branch 8 that can be releasably connected to an oxygen source, such as a portable pressure vessel or an oxygen circuit provided within an health care facility such as a hospital and the exhalation branch 4 comprises an air treating unit with a trap 9 for carbon dioxide and preferably also an antiviral / antibacterial filter i.e. antimicrobial 10.

In order to adjust the pressure inside the circuit during the user's breathing cycles and therefore, as clinically tested, improve the absorption of oxygen in the lungs, circuit 2 includes at least one accumulator C, e.g. of 1 liter, preferably one per branch 3, 4. The effect of the accumulator C can also be obtained by an appropriate sizing of the internal volume of the circuit, e.g. diameter and length of the tubes and internal volume of the wearable breathing element 5, especially when a helmet is used.

In order to prevent any high microbial load air leaks towards the outside, which is harmful to those around the user, circuit 2 comprises at least one antimicrobial filter 10a preferably arranged at the outlet of the wearable breathing element 5, even more preferably downstream of the non-return valve 7. According to a preferred embodiment, further ports towards the outside of circuit 2, preferably all of them, comprise a respective antimicrobial filter. In particular, the circuit in the figure includes a vent port 11 and the derivations 8 for the entry of oxygen and 12 for the entry or exit of pressurized air. Circuit 1 comprises, downstream of trap 9, a gas analysis device 13, for example electronic. The related data allow monitoring of the user's conditions, in particular of pressure and of one or more of other chemical or physical parameters such as flow rate, temperature, relative humidity, quantity of oxygen and carbon dioxide. For example, the gas analysis device is of the type usable in an operating room.

According to a preferred embodiment, circuit 1 is associated with an electronic control unit 15 connected in data exchange with gas analysis device 13 and programmed to perform one or more of the following functions:
- Monitoring one or more physical and / or chemical parameters of the gas and generate warning messages when these parameters reach or exceed predefined thresholds. Such thresholds can be static, e.g. constant over time and adaptable e.g. through the electronic control unit 15 before using the circuit; or adaptive or dynamic e.g. vary during use on the basis of mathematical models stored in the electronic control unit 15;
- Actuating one or more actuated valves 16 on the basis of one or more signals from the gas analysis device 13. For example, if the pressure in circuit 2 during use is too low or too high, valve 16 relating to port 12, i.e. a pressure regulating valve as shown in the figure, to fill-in or evacuate air and restore a condition of operational use of the circuit. Preferably, the evacuation of air through inlet 12 is performed to maintain the CPAP pressure, without the intervention of safety valve 16 associated with vent port 11, the valve being calibrated at a pressure higher than the operating CPAP pressure. In particular, to supplement the effect of accumulators C, the transit of gas e.g. air through port 12 to the circuit occurs during inspiration and the transit of gas to the outside of the closed circuit occurs during exhalation; or, when too high values of carbon dioxide are detected, generating a signal for the replacement or washing of trap 9; or adjusting the amount of oxygen entering through branch 8 and / or air through port 12. This happens to keep the CPAP pressure at its substantially constant value during the user's breathing and, at the same time, the desired value of FiO₂ on the basis of a specific measurement of the internal pressure of the closed circuit. The valves can therefore be either discretely valves, e.g. for vent port 11, or continuous valves, with or without feedback for the position of the shutter, as in the case of branch 11 and port 12 for the purpose of oxygen / air dosing; or the quantity of oxygen in the circuit; or open the vent port in case of excessive temperature and / or humidity.

For example, gas analysis e.g. exhaled air is performed by photoacoustic spectrometry, refractometry, piezoelectric absorption, Raman scattering, mass spectrometry.

In use, the circuit is connected to a source of pressurized air and oxygen in such a way that, preferably, a gas mixture consisting of nitrogen and oxygen circulates in the circuit, i.e. the user breathes air. According to an embodiment, the source of pressurized air is a centrifugal fan having a controlled rotational speed, which in conditions of zero average flow rate of air in the circuit is able to maintain the constant value of CPAP pressure at the delivery. Furthermore, even in the presence of a centrifugal fan, the air is evacuated from the inside of the circuit towards the outside to reduce the pressure when the internal pressure of the circuit exceeds the delivery pressure generated at an assigned number of revolutions and in condition of zero flow. Since a centrifugal fan has for each angular speed value a corresponding value of delivery pressure at zero flow, the electronic control unit 15 is programmed to apply the number of revolutions of the centrifugal fan for which the delivery pressure at zero flow is equal to the desired CPAP pressure.

Alternatively, it is possible to provide a bellows connected to port 12, to which a calibrated weight of constant mass applied to the head of the bellows itself is applied to obtain constant CPAP pressure in the closed circuit. In this case, of particularly simple execution, the constant positive pressure value is obtained by compensating the volumes of air inhaled or exhaled, even abruptly, by simple weight oscillation. In fact, the pressure value is exclusively determined by the action of the weight, whose constant mass leads to a constant pressure value in the closed circuit. In this embodiment, however, a controlled pressure regulating valve 16 or another controllable source of pressurized air may be present to supplement or evacuate any volume of air which tend to cause a reduction or increase in the CPAP pressure in the closed circuit and / or to introduce into the circuit a quantity of fresh washing air at CPAP pressure.

According to an exemplary embodiment, via the control unit 15, the pressure inside the circuit during use does not exceed 25 cmH₂O, preferably does not exceed 20 cmH₂O: this value is sufficient to facilitate the opening of the pulmonary alveoli during inspiration and, at the same time, can be obtained by means of a fan, also battery operated and preferably centrifugal, as well as by means of a pressurized air circuit controlled by the controlled pressure regulating valve 16 in order to obtain the desired positive and constant pressure. Likewise, branch 8 can be connected to an oxygen pressure vessel or to a circuit of a facility, including a field, hospital or first aid facility. It is therefore possible to size a kit including circuit 2 and the sources of pressurized air and oxygen so as to be portable and operable at least for predefined time intervals even without being connected to power supply grids: this allows use even in remote areas, impervious or with damaged systems. Furthermore, the minimum value of the constant positive pressure within the circuit is 5 cmH₂O and this is achieved via the use of the gas analysis device 13 including a pressure sensor. Control unit 15, on the basis of the signal from the pressure sensor, controls the source of pressurized gas e.g. the fan by means of the relative number of revolutions or the controlled pressure regulating valve 16 when the derivation 12 is connected to a generic source of pressurized air; and controls the reintegration of only the amount of oxygen consumed by the user through the flow control valve 16 associated with branch 8.

In use, when the wearable breathing element is a helmet and the leaks are ideally zero, the inlet and outlet flow rates of air or nitrogen through branch 12 are at zero average values since the quantity of inhaled gas and that of gas exhaled is normally the same and, in the case of high or ideally infinite circuit capacity, the flow to branch 12 is zero. However, it is possible to provide for a washing step, for example at regular intervals of programmable periodicity via the control device 15, in which appropriately pressurized fresh atmospheric air is introduced at the CPAP positive pressure to replace the recirculating gas mixture.

If during use for long periods, small gas leaks from the circuit to the external environment occur, e.g. through the seals of the breathing wearable element 5, the pressure sensor detects a drop in the internal pressure of the circuit and, through appropriate adjustment of the source of pressurized air by the control unit 15, a quantity of new gas would be added, preferably air, to restore the constant pressure inside the circuit to the desired value during the user's autonomous breathing. Such additions of air volumes are however absent in the ideal case of a closed circuit without leaks and with high or ideally infinite capacity.

Circuit 2 was built in prototype form using existing devices. The main purpose of this demonstration circuit is to verify the actual performance of the circuit. Three blowers for the home treatment of sleep apnea in CPAP mode were tested together with two anesthetic gas analyzers and two different sources of oxygen. Reference CPAP pressure values of 5, 10, 15 and 20 cm H2O were applied. All measurements were collected 5 minutes after operational setup in order to achieve stabilization of the oxygen concentration within the circuit. The respiratory rate of a healthy human volunteer ranged from 10 to 14 breaths per minute. The test results are summarized in Table 1. For all the above reference pressure settings, the flow rate was always less than 10 L / min. The incomplete tightness of the CPAP helmet was verified, which required oxygen flow rates greater than those strictly necessary to compensate for the patient's consumption. In an industrialized version of the circuit, the wearable breathing element will be configured to be hermetic when worn: for example through a specific design of the sealing systems, e.g. elastic silicone valves, on the patient's body. The resistance to the air flow of the circuit components is always less than 0.5 cmH₂O, measured by imposing the maximum recorded flow rate of 10 l / min. Furthermore, the noise both in the helmet and in the environment was negligible.

The carbon dioxide produced was totally absorbed by the soda lime of trap 9, as reported in the inspiratory data column of Table 1 (iCO₂, EtCO₂: inhaled, exhaled CO₂). In all cases the fan was able to set the rpm values such to keep the CPAP pressure in the entire range of 5-20 cmH2O thanks to the measurement of the pressure through the gas analysis device 13 and the connected control unit 15 both to the device 13 and to the blower.

**Table 1**

| **CPAP** [cmH₂O] | **O₂** [l/mm] (Cylinder) | **O₂** [l/mm] (Concentr.) | **FiO₂** helmet [%] | **iCO₂** hehnet [Torr] | **FiO₂** Exp. limb [%] | **EtCO₂** Exp. limb [Torr] |
|---|---|---|---|---|---|---|
| 5 | 1 | (1) | 40 (40) | 0 | 33 (33) | 23 |
| 10 | 1 | (1) | 35 (33) | 0 | 32 (31) | 20 |
| 15 | 1 | (1) | 31 (30) | 0 | 29 (27) | 18 |
| 20 | 1 | (1) | 27 (26) | 0 | 26 (25) | 14 |
| 5 | 2 | (-) | 62 | 0 | 48 | 24 |
| 10 | 2 | (-) | 61 | 0 | 47 | 23 |
| 15 | 2 | (-) | 57 | 0 | 46 | 18 |
| 20 | 2 | (-) | 36 | 0 | 36 | 16 |
| 10 | 4 | (-) | 80 | 0 | 66 | 21 |

The test results confirmed that the closed configuration of circuit 1 guarantees effective control of the air to be breathed and of its high fraction of oxygen, obtained with a very low oxygen supply, about two orders of magnitude less than in conventional CPAP open circuits, thus avoiding the waste of medical gases. Tests have also shown that the proposed closed-loop configuration ensures high efficiency. In fact, FiO₂ values are obtained, i.e. percentage of oxygen in the inspired air, of 30-40% with only 1 1 / min of oxygen. This low oxygen flow allows for the potential replacement of the medical oxygen cylinder with an oxygen concentrator.

Measured FiO₂ levels were highest when measured proximal to the breathing wearable 5 to the expiratory branch, confirming the correct direction of flow in the circuit.

The data reported on the expiratory CO₂ measurements were lower than the standard physiological values due to the dilution effect of the dead space of the helmet, which acts as an accumulator, and, for the same reason, decreased almost linearly with increasing pressure levels. CPAP.

Finally, it is clear that it is possible to apply modifications or variants to the kit described and illustrated here without departing from the scope of protection as defined by the attached claims.

For example, it is possible to insert a safety valve with automatic opening calibrated with respect to a maximum pressure whose actuation is independent of the pressure sensor. This valve on the diagram could be integrated into the controlled valve 16 associated with the vent port 11.

## Claims

1. Breathing support kit comprising: a first inlet (12) for receiving pressurized air; a second inlet (8) for receiving oxygen; a section where the air coming from the first entrance and the oxygen coming from the second entrance converge; an air circuit (2) comprising the section, a partial or full face mask as a wearable breathing element (5) applicable at least on the nose and mouth of a patient and configured to receive from the circuit a mixture of air and oxygen coming from said section through a first circuit branch (3) and to transfer to the circuit an enriched mixture of exhaled air comprising carbon dioxide in a second circuit branch (4), an air treatment filter (9) along the second circuit branch to reduce the quantity of carbon dioxide, a valve assembly (6, 7) configured to impose air circulation in a single direction from the section to reach the treatment filter (9) through the breathing wearable element (5), the first and second branches (3, 4) being fluidically connected to each other to define in use a closed air circuit, a pressure sensor (13) and a pressure regulation device (16) configured to adjust the pressure within the closed circuit based on a signal from the pressure sensor (13) to obtain a CPAP pressure during the spontaneous breathing of the user in the breathing wearable element (5), wherein the pressure regulating device (15, 16) is configured to obtain a CPAP pressure between 10 cmH₂O and 25 cmH₂O, preferably between 10 and 20 wherein the circuit (2) further comprises a gas analysis device (13) configured to detect one or more physical and / or chemical parameters of the circulating air, including said pressure sensor, and a control unit (15) programmed to generate an alert or alarm signal based on one or more signals from the gas analysis device (13) and to control said pressure regulation device (16).

2. Kit according to claim 1, wherein the pressure regulating device (16) is configured to treat a gas mix constituted of nitrogen and oxygen to obtain a predefined FiO₂ value.

3. Kit according to any of the previous claims, wherein in at least a functioning condition, a flow of nitrogen or air entering the closed circuit via said inlet (12) is zero during a breathing cycle of the user and oxygen is added to obtain a predefined FiO₂ value.

4. Kit according to claim 1, wherein the valve assembly (6, 7) is configured to open and close to conduct air in the direction of circulation following the respiratory activity of the patient to whom the breathing wearable element (5) is applied.

5. Kit according to any of the preceding claims, comprising a capacity tank (C) connected in derivation to the first branch to reduce the pressure fluctuations of the air entering the wearable breathing element (5).

6. Kit according to any of the previous claims, wherein the control unit (15) is programmed to open a vent valve (11) based on one or more signals of the gas analysis device (13) or the vent valve is opens automatically when a predetermined level of pressure in the circuit is reached.

7. Kit according to any of the preceding claims, wherein the circuit (2) comprises an antiviral/antibacterial or antimicrobic filter (10) to purify the circulating air.

8. Kit according to any one of the preceding claims, comprising a pressurized air generator releasably connected to said first inlet (12).

9. Kit according to claim 8, wherein the pressurized air source comprises an electric centrifugal fan.

10. Kit according to claim 8, wherein the pressurized air source comprises a bellows and a weight on the bellows to apply a compression on the gas volume inside the closed circuit.

11. Kit according to any one of the preceding claims, comprising an oxygen source releasably connected to said second inlet (8).

## Patentansprüche

1. Beatmungsgerät bestehend aus: einem ersten Einlass (12) zur Aufnahme von Druckluft ; einem zweiten Einlass (8) zur Aufnahme von Sauerstoff; einem Abschnitt, in dem die aus dem ersten Einlass kommende Luft und der aus dem zweiten Einlass kommende Sauerstoff zusammenlaufen; einem Luftkreislauf (2) umfassend den Abschnitt, einer Teil-oder Vollgesichtsmaske als ein tragbares Atemelement (5), das mindestens an Nase und Mund eines Patienten angebracht werden kann und so konfiguriert ist, dass es aus dem Kreislauf über einen ersten Kreislaufzweig (3) ein Gemisch aus Luft und Sauerstoff aus dem Abschnitt aufnimmt und über einen zweiten Kreislaufzweig (4) ein mit Kohlendioxid angereichertes Gemisch aus Ausatemluft in den Kreislauf überträgt, einem Luftreinigungsfilter (9) entlang des zweiten Kreislaufzweigs zur Reduzierung der Kohlendioxidmenge, einer Ventilanordnung (6, 7), die so konfiguriert ist, dass eine Luftzirkulation vom Abschnitt über das tragbare Atemelement (5) zum Reinigungsfilter (9) in eine Richtung erfolgt, wobei der erste und der zweite Zweig (3, 4) durch Fluidverbindung miteinander verbunden sind, um im Gebrauch einen geschlossenen Luftkreislauf zu definieren, einem Drucksensor (13) und einer Druckregelungseinrichtung (15, 16), die so konfiguriert sind, dass sie den Druck innerhalb des geschlossenen Kreislaufs anhand eines Signals des Drucksensors (13) anpassen, um während der Spontanatmung des Benutzers im tragbaren Atemelement (5) einen CPAP-Druck zu erreichen, wobei die Druckregelungsvorrichtung (15, 16) derart konfiguriert ist, um einen CPAP-Druck zwischen 10 cmH₂O und 25 cmH₂O, vorzugsweise zwischen 10 und 20, wobei der Kreislauf weiter ein Gasanalysegerät (13) umfasst, das so konfiguriert ist, dass es einen oder mehrere physikalische und/oder chemische Parameter der zirkulierenden Luft erfasst, sowie eine Steuereinheit (15), die so programmiert ist, dass sie auf der Grundlage eines oder mehrerer Signale des Gasanalysegeräts (13) ein Warn- oder Alarmsignal erzeugt und die Druckregelungsvorrichtung (16) steuert.

2. Kit nach Anspruch 1, wobei die Druckregelungsvorrichtung (16) so konfiguriert ist, dass sie ein aus Stickstoff und Sauerstoff bestehendes Gasgemisch aufbereitet, um einen vordefinierten FiO₂-Wert zu erreichen.

3. Kit nach einem der vorhergehenden Ansprüche, wobei in mindestens einem funktionsfähigen Zustand der Stickstoff- oder Luftstrom, der über den Einlass (12) in den geschlossenen Kreislauf eintritt, während eines Atemzyklus des Benutzers null ist und Sauerstoff hinzugefügt wird, um einen vordefinierten FiO₂-Wert zu erreichen.

4. Kit nach Anspruch 1, wobei die Ventilanordnung (6, 7) so konfiguriert ist, dass sie sich öffnet und schließt, um Luft in Richtung des Kreislaufs entsprechend der Atemaktivität des Patienten zu leiten, an dem das tragbare Atemelement (5) angebracht ist.

5. Kit nach einem der vorhergehenden Ansprüche, umfassend einen Vorratsbehälter (C), der in Ableitung mit dem ersten Zweig verbunden ist, um die Druckschwankungen der in das tragbare Atemelement (5) einströmenden Luft zu reduzieren.

6. Kit nach einem der vorhergehenden Ansprüche, wobei die Steuereinheit (15) so programmiert ist, dass sie ein Entlüftungsventil (11) auf der Grundlage eines oder mehrerer Signale des Gasanalysegeräts (13) öffnet, oder dass sich das Entlüftungsventil automatisch öffnet, wenn ein vorbestimmter Druckpegel im Kreislauf erreicht wird.

7. Kit nach einem der vorhergehenden Ansprüche, wobei der Kreislauf (2) einen antiviralen/antibakteriellen oder antimikrobiellen Filter (10) umfasst, um die zirkulierende Luft zu reinigen.

8. Kit nach einem der vorhergehenden Ansprüche, umfassend einen Drucklufterzeuger, der lösbar mit dem ersten Einlass (13) verbunden ist.

9. Kit nach Anspruch 8, wobei die Druckluftquelle ein elektrischer Radialventilator umfasst.

10. Kit nach Anspruch 8, wobei die Druckluftquelle einen Balg und ein Gewicht auf dem Balg umfasst, um eine Kompression auf das Gasvolumen innerhalb des geschlossenen Kreislaufs auszuüben.

11. Kit nach einem der vorhergehenden Ansprüche, umfassend eine Sauerstoffquelle, die lösbar mit dem zweiten Einlass (8) verbunden ist.

## Revendications

1. Kit d'assistance respiratoire comprenant : une première entrée (12) pour recevoir de l'air comprimé ; une deuxième entrée (8) pour recevoir de l'oxygène ; une section où convergent l'air provenant de la première entrée et l'oxygène provenant de la deuxième entrée ; un circuit d'air (2) comprenant la section, un masque partiel ou complet comme un élément respiratoire portable (5) applicable au moins sur le nez et la bouche d'un patient et configuré pour recevoir du circuit un mélange d'air et d'oxygène provenant de ladite section par l'intermédiaire d'une première branche de circuit (3) et pour transférer au circuit un mélange enrichi d'air expiré comprenant du dioxyde de carbone dans une deuxième branche de circuit (4), un filtre de traitement de l'air (9) le long de la deuxième branche de circuit pour réduire la quantité de dioxyde de carbone, un ensemble de vannes (6, 7) configuré pour imposer une circulation d'air dans une seule direction de la section pour atteindre le filtre de traitement (9) à travers l'élément respiratoire portable (5), les première et deuxième branches (3, 4) étant connectées fluidement l'une à l'autre pour définir en utilisation un circuit d'air fermé, un capteur de pression (13) et un dispositif de régulation de pression (15, 16) configurés pour ajuster la pression à l'intérieur du circuit fermé en fonction d'un signal provenant du capteur de pression (13) afin d'obtenir une pression CPAP pendant la respiration spontanée de l'utilisateur dans l'élément respiratoire portable (5), dans lequel le dispositif de régulation de pression (15, 16) est configuré pour obtenir une pression CPAP entre 10 cmH₂O et 25 cmH₂o, de préférence entre 10 et 20, dans lequel le circuit (2) comprend en outre un dispositif d'analyse de gaz (13) configuré pour détecter un ou plusieurs paramètres physiques et/ou chimiques de l'air en circulation, comprenant ledit capteur de pression, et une unité de commande (15) programmée pour générer un signal d'alerte ou d'alarme en fonction d'un ou plusieurs signaux provenant du dispositif d'analyse de gaz (13) et pour commander ledit dispositif de régulation de pression (16).

2. Kit selon la revendication 1, dans lequel le dispositif de régulation de pression (16) est configuré pour traiter un mélange gazeux constitué d'azote et d'oxygène afin d'obtenir une valeur prédéfinie de FiO₂.

3. Kit selon l'une quelconque des revendications précédentes, dans lequel, au moins en condition de fonctionnement, un flux d'azote ou d'air entrant dans le circuit fermé via ladite entrée (12) est nul pendant un cycle respiratoire de l'utilisateur et de l'oxygène est ajouté pour obtenir une valeur prédéfinie de FiO₂.

4. Kit selon la revendication 1, dans lequel l'ensemble de vannes (6, 7) est configuré pour s'ouvrir et se fermer afin de conduire l'air dans la direction de circulation suivant l'activité respiratoire du patient auquel l'élément respiratoire portable (5) est appliqué.

5. Kit selon l'une quelconque des revendications précédentes, comprenant un réservoir de capacité (C) connecté en dérivation à la première branche pour réduire les fluctuations de pression de l'air entrant dans l'élément respiratoire portable (5).

6. Kit selon l'une quelconque des revendications précédentes, dans lequel l'unité de commande (15) est programmée pour ouvrir une vanne de ventilation (11) sur la base d'un ou plusieurs signaux du dispositif d'analyse de gaz (13) ou la vanne de ventilation s'ouvre automatiquement lorsqu'un niveau de pression prédéterminé dans le circuit est atteint.

7. Kit selon l'une quelconque des revendications précédentes, dans lequel le circuit (2) comprend un filtre antiviral/antibactérien ou antimicrobien (10) à fin de purifier l'air en circulation.

8. Kit selon l'une quelconque des revendications précédentes, comprenant un générateur d'air pressurisé relié de manière amovible à ladite première entrée (12).

9. Kit selon la revendication 8, dans lequel la source d'air pressurisé comprend un ventilateur centrifuge électrique.

10. Kit selon la revendication 8, dans lequel la source d'air pressurisé comprend un soufflet et un poids sur le soufflet pour appliquer une compression sur le volume de gaz à l'intérieur du circuit fermé.

11. Kit selon l'une quelconque des revendications précédentes, comprenant une source d'oxygène reliée de manière amovible à ladite deuxième entrée (8).
